# EUROPEAN PATENT SPECIFICATION

(11) **EP 2 330 896 B1**
(45) Date of publication and mention of the grant of the patent: **25.11.2015**
(21) Application number: 09812773.1
(22) Date of filing: 06.09.2009
(51) Int. Cl.: A61K 31/70, A61K 38/57, A61P 21/00

(54) **ANTINECROTIC ACTIVITY OF ALPHA 1-ANTITRYPSIN**
ANTINEKROTISCHE WIRKUNG VON ALPHA 1-ANTITRYPSIN
ACTIVITÉ ANTINÉCROTIQUE D'ALPHA 1-ANTITRYPSINE

(30) Priority: 10.09.2008 US 129707 P
(43) Date of publication of application: 15.06.2011
(73) Proprietor: Ben Gurion University Of The Negev Research And Development Authority, 84105 Beer Sheva (IL); Mor-Research Applications Ltd., 69710 Tel Aviv (IL)
(72) Inventor: NATHAN, Ilana, 84965 Omer (IL); LEWIS, Eli, 84750 Beer Sheva (IL); KHALFIN, Boris, 80500Yeruham (IL)
(74) Representative: Modiano, Micaela Nadia
(86) International application number: PCT/IL2009/000861
(87) International publication number: WO 2010/029537

(56) References cited:
- WO-A2-00/51623
- WO-A2-00/51624
- WO-A2-03/028621
- US-A1- 2003 053 998
- US-A1- 2007 224 671
- US-B1- 6 262 020
- GILUTZ H ET AL: "ALPHA-1 ANTI TRYPSIN IN ACUTE MYO CARDIAL INFARCTION", BRITISH HEART JOURNAL, BRITISH MEDICAL ASSOCIATION, LONDON, GB, vol. 49, no. 1, 1 January 1983 (1983-01-01), pages 26-29, XP009163182, ISSN: 0007-0769
- BARTOSIK-PSUJEK H ET AL: "Markers of inflammation in cerebral ischemia", NEUROLOGICAL SCIENCES (TESTO STAMPATO), SPRINGER VERLAG, MILAN, IT, vol. 24, no. 4, 1 November 2003 (2003-11-01), pages 279-280, XP009163174, ISSN: 1590-1874
- REQUENA ET AL: "Normal Subcutaneous Fat, Necrosis of Adipocytes and Classification of the Panniculitides", SEMINARS IN CUTANEOUS MEDICINE AND SURGERY, W.B. SAUNDERS, PHILADELPHIA, US, vol. 26, no. 2, 1 June 2007 (2007-06-01), pages 66-70, XP022096251, ISSN: 1085-5629, DOI: 10.1016/J.SDER.2007.02.001
- OHO ET AL.: 'Increased elastin-degrading activity and neointimal formation in porcine aortic organ culture. Reduction of both features with a serine proteinase inhibitor' ATHEROSCLER. THROMB. VASC. BIOL., [Online] vol. 15, no. 12, December 1995, pages 2200 - 2206, XP008145740 Retrieved from the Internet: <URL:http://atvb.ahajournals.org/cgi/conten t/full/15/12/2200> [retrieved on 2009-12-28]

## Description

### BACKGROUND OF THE INVENTION

Necrosis is considered to be a unique process of death of cells and living tissue, distinguished from apoptotic programmed cell death, Necrosis is characterized by cell swelling, chromatin digestion, and disruption of the plasma and organelle membranes. Latter stages of necrosis are characterized by extensive DNA hydrolysis, vacuolation of the endoplasmic reticulum, organelle breakdown, and cell lysis. The release of intracellular contents after plasma membrane rupture is the cause of inflammation seen with necrosis. Necrosis has long been viewed as an accidental pathological mode of cell death. Recent studies have presented severalines of evidence indicating that necrosis is a regulated process.

In contrast to apoptosis, cleanup of cell debris by phagocytes of the immune system is generally more difficult, as the regulated necrotic pathway generally does not provide specific cell signals for resident or recruited phagocytes to dispose of the necrotic cells and byproducts thereof. The immune system, as a consequence of the lack of appropriate specific signals is less capable of locating necrotic cells and tissue and thereby disposing of the noxious products.

There are many causes of necrosis including prolonged exposure to injury, infection, cancer, infarction, poisons, venoms and inflammation. Necrosis can arise from lack of proper care to a wound site.

Necrosis also plays a part in the pathology of several severe diseases including myocardial infarction, brain stroke, liver cirrhosis and other potentially lethal diseases. Several existing therapies for necrosis, such as early and aggressive surgical debridement and exploration of necrotic tissue, hyperbaric oxygen therapy, administration of antibiotics, anti-inflammatory drugs and intravenous immunoglobulin are used with mixed success.

U.S. Application Publication No. 2003/0053998 discloses method of protecting organs or tissue susceptible to reperfusion-induced dysfunction alter ischemia, by parenterally administering to a patient a pharmaceutical composition containing natural alpha-1 acid glycoprotein, natural alpha-1 antitrypsin or a mixture thereof. U.S. Application Publication No. 2003/0053998 also discloses that organ or tissue transplants can be contacted with natural alpha-1 acid glycoprotein, natural alpha-antitrypsin or mixtures by perfusing or flushing them with a solution containing natural alpha-1 acid glycoprotein, natural alpha-1 antitrypsin or mixtures thereof in a concentration of 0.1 to 5 g/l.

Gilutz et al., (Gilutz H. et al., 1983. British Heart J., British Med. Assoc. 49(I):26-29 describes the presence and level of alpha-1 antitrypsin (AAT) in acute myocardial infarction. Their findings suggest that the course of myocardial infarction is determined not only by the severity of the ischemic event, but also by the response of the "acute phase reaction" mechanism. It is thus concluded that a failure of AAT levels to increase after myocardial infarction may be associated with a worse clinical course.

An ideal treatment for inhibiting and/or treating necrosis is unavailable and a significant morbidity and mortality is attributable to complications of necrosis.

### SUMMARY OF THE INVENTION

The present invention refers to the use of a therapeutically effective amount of alpha-1-antitrypsin or an active homologue or an active variant thereof for the preparation of a medicament for treatment of tissue necrosis, wherein the medicament is to be administered to a tissue characterized by undergoing necrosis and wherein the effective amount of alpha-1-antitrypsin inhibits the necrosis within the tissue.

The present invention also relates to a method for inhibiting necrosis in a tissue culture, said method comprising contacting a tissue culture characterized by undergoing necrosis with an amount of alpha-1-antitrypsin sufficient to inhibit necrosis in said tissue culture.

The present invention furhter relates to a method for preventing necrosis in a tissue culture, said method comprises contacting a tissue culture characterized by undergoing necrosis with an amount of alpha-1-antitrypsin sufficient to prevent necrosis in said tissue culture.

In one embodiment, this disclosure comprises a method of treating a subject suffering from a disease characterized by tissue necrosis, said method comprising administering a therapeutically effective amount of alpha- 1-antitrypsin or a homologue or variant thereof to said subject, wherein the effective amount inhibits said tissue necrosis and said disease is characterized in that affected tissue is undergoing necrosis as opposed to apoptosis. In one embodiment, at least 51% of affected tissue in said subject is undergoing necrosis as opposed to apoptosis.

In one embodiment, this disclosure further comprises a method of prophylactically treating a subject at risk for a pathological condition that is precipitated at least in part by tissue necrosis, said method comprising: administering to said subject a therapeutically effective amount of alpha-1-antitrypsin such that said effective amount inhibits tissue necrosis in said subject.

In one embodiment, this invention comprises a method for inhibiting necrosis in a cell or tissue culture, for example such that takes place before cell or tissue transplant (stem cells, skin), said method comprising contacting a cell or tissue in culture with an amount of alpha-1-antitrypsin sufficient to inhibit necrosis in said cell or tissue in culture.

### BRIEF DESCRIPTION OF THE FIGURES

Figure 1. A bar graph showing that Alpha-1-antitrypsin (AAT) causes a stable decrease in the LDH release in U-937 cells which occurs after induction of necrosis by incubation with KCN for seven hours is presented in 1A. The effect of AAT on PC 12 cells treated with oligomycin - anti-Fas induced cell death as assessed by the determination of LDH release is presented in 1B.
Figure 2. Is a bar graph: an increase in surviving of U-937 cells due to alpha-1-antitrypsin treatment after induction of necrosis by incubation with KCN for seven hours assayed by trypan blue exclusion.
Figure 3. Is a bar graph: a decrease in the percent of necrotic U-937 cells achieved by pre-incubation with alpha-1-antitrypsin for 30 min followed by incubation with KCN for seven hours assayed by acridine orange/ethydium bromide dual staining.
Figure 4. Is a bar graph: a stable decrease in the LDH release in PC-12 cells was recorded when the cells were maintained in glucose-free medium, pre-incubated with or without Alpha-1-antitrypsin for 30 min and then KCN was added for five hours.
Figure 5. Is a bar graph: showing the effect of AAT on KCN-induced necrosis in PC 12 cells (% survival). Cells were maintained in glucose-free medium, pre-incubated with or without AAT for 30 min and then KCN was added for five hours. Thereafter alive cells were stained and counted by trypan blue exclusion.
Figure 6. A bar graph showing serum pancreatic lipase levels in controls and in treated groups is presented in 6A. A graph presenting morbidity assessment on a daily basis in controls and in treated groups is presented in 6B. A micrograph of macroscopic inspection of pancreata from AAT-treated ligated animals, animals with healthy pancreata, and control, untreated animals, is displayed in 6C.

### DETAILED DESCRIPTION OF THE INTENTION

This disclosure provides in one embodiment a method for treatment of a subject suffering from tissue necrosis. The method comprises of administering a therapeutically effective amount of Alpha-1-antitrypsin (AAT) or a homologue or variant thereof to said subject, wherein the effective amount inhibits said tissue necrosis and said disease is characterized in that affected tissue in the subject is undergoing necrosis as opposed to apoptosis. In another embodiment, a therapeutically effective amount of AAT is administered in a composition. In another embodiment, a therapeutically effective amount of AAT is administered in a pharmaceutical composition. In some embodiments, the disease is characterized in that at least 51 % of affected tissue in the subject is undergoing necrosis as opposed to apoptosis. The disclosure further comprises a method of prophylactically treating a subject at risk for a pathological condition that is precipitated at least in part by tissue necrosis, by administering to said subject a therapeutically effective amount of alpha-1-antitrypsin such that the effective amount inhibits tissue necrosis in subject. The invention comprises a method for inhibiting necrosis in a cell or tissue culture, comprising contacting a cell or tissue in culture with an amount of alpha-1-antitrypsin sufficient to inhibit necrosis in the cell or tissue in culture,

Necrosis, cell death or tissue death is one of the pathologies seen in several diseases. For example in diabetes, open wounds which are not treated may result in the development of necrosis. When cells or a tissue do not receive oxygen for a prolonged period of time, necrotic cells death occurs. This is evident in cardiac infarction and in stroke, where the related tissue is demonstrably affected.

Another form of necrosis is aseptic necrosis which is bone death caused by poor blood supply to the area. It is most common in the hip, knee, and shoulder. Aseptic necrosis occurs when at least part of a bone is poorly perfused, Under such circumstances, part(s) of the bone fractures. If this condition is not treated, bone damage worsens, and remaining healthy/unaffected regions of the bone may collapse.

Another form of necrosis arises from dead tissue formation at a site of radiation. This is called radiation necrosis which forms from radiation cancer therapy. In some aspects, the mass of dead tissue contains both cancerous and healthy cells. Radiation necrosis can develop over a period of months to years, providing a reasonable venue for prophylactic treatment of such patients. This necrotic process may result in dementia, headache and seizures. It is not always easy to tell the difference between radiation necrosis and cancer that has come back. Analysis by PET scan, can sometimes tell the difference between dead tissue and living cancer tissue, but often a biopsy is the only way to precisely determine necrosis.

In one embodiment, a method for increasing cell viability in a necrotic tissue. In one embodiment, a method for increasing cell viability in a pre-necrotic tissue. In one embodiment, a method for protecting a cell against necrosis induced by a necrosis inducing agent is provided. In another embodiment, necrosis inducing agent is an endogenic factor or an exogenic factor.

A more common, yet still rare form of necrosis is necrotizing soft-tissue infection which is a severe type of tissue infection that can involve the skin, subcutaneous fat, the muscle sheath (fascia), and the muscle. It can cause gangrene, tissue death, systemic disease and death. Necrotizing subcutaneous infection or fasciitis can be caused by a variety of bacteria including oxygen-using bacteria (aerobic) or oxygen-avoiding bacteria (anaerobic). This type of infection develops when bacteria enter the body, usually through a minor skin injury or abrasion. The bacteria begin to grow and release toxins that directly kill tissue, interfere with the blood flow to the tissue, digest materials in the tissue, which rapidly spreads the bacteria and cause widespread effects, such as shock: The appearance of the skin and underlying tissues, and the presence of gangrene (black or dead tissue) indicate a necrotizing soft tissue infection. Imaging tests, such as CT scans, are sometimes helpful. Powerful, broad-spectrum antibiotics must be given immediately through a vein (IV). This is an attempt to control the infection by quickly raising the blood levels of the antibiotic. Surgery is required to open and drain infected areas and remove dead tissue. Skin grafts may be required after the infection is cleared. If the infection is in a limb and cannot be contained or controlled, amputation of the limb may be considered. Sometimes pooled immunoglobulins (antibodies) are given by vein to help fight the infection. If the organism is determined to be an oxygen-avoiding bacteria (anaerobe), the patient may be placed in a hyperbaric oxygen chamber, a device in which the patient is given 100% oxygen at several atmospheres of pressure.

Outcomes are variable. The type of infecting organism, rate of spread, susceptibility to antibiotics, and the timing of diagnosis all contribute to the final outcome. Scarring and deformity are common with this type of disease. Fatalities are high even with aggressive treatment and powerful antibiotics. Untreated, the infection invariably spreads and causes death.

In another embodiment, the disclosure provides a method of preventing pancreatitis in a subject, comprising administering to a subject at risk of being afflicted with pancreatitis a therapeutically effective amount of alpha-1-antitrypsin, thereby preventing pancreatitis in a subject. In another embodiment, the disclosure provides a method of reducing the severity of pancreatitis in a subject, comprising administering to a subject a therapeutically effective amount of alpha-1-antitrypsin. In another embodiment, the disclosure provides a method of reducing the symptoms associated with of pancreatitis in a subject, comprising administering to a subject a therapeutically effective amount of alpha-1-antitrypsin. In another embodiment, the disclosure provides a method of treating pancreatitis in a subject, comprising administering to a subject a therapeutically effective amount of alpha-1-antitrypsin. In another embodiment, the disclosure provides a method of curing pancreatitis in a subject, comprising administering to a subject a therapeutically effective amount of alpha-1-antitrypsin. In another embodiment, the disclosure provides a method of ameliorating pancreatitis in a subject afflicted with pancreatitis, comprising administering to a subject a therapeutically effective amount of alpha-1-antitrypsin. In another embodiment, the disclosure provides a method of improving the wellbeing of a subject afflicted with pancreatitis, comprising administering to a subject a therapeutically effective amount of alpha-1-antitrypsin.

In another embodiment, the disclosure provides a method of preventing pancreatitis in a subject, comprising administering to the subject a therapeutically effective amount of alpha-1-antitrypsin prior to an abdominal surgical procedure in said subject, thereby preventing pancreatitis in a subject. In another embodiment, the abdominal surgical procedure is endoscopic retrograde cholangiopancreatography (ERCP), pancreatic stenting, pancreaticoduodenectomy, pancreatectomy, or any combination thereof.

In another embodiment, preventing pancreatitis in a subject further comprises reducing the risk of pancreatitis. In another embodiment, preventing pancreatitis in a subject further comprises reducing the severity of pancreatitis.

In another embodiment, the disclosure provides a method of preventing pancreatitis in a subject, comprising administering to the subject a therapeutically effective amount of alpha-1-antitrypsin in combination with an additional active pharmaceutical ingredient prior to an abdominal surgical procedure in said subject.

In another embodiment, the disclosure provides a method of preventing pancreatitis induced by a pancreatitis causing medicine in a subject, comprising administering to the subject a therapeutically effective amount of alpha-I-antitrypsin prior to and/or during the treatment with a pancreatitis causing medicine. In another embodiment, a pancreatitis causing medicine is an AIDS drug. In another embodiment, a pancreatitis causing medicine is a DDL In another embodiment, a pancreatitis causing medicine is pentamidine. In another embodiment, a pancreatitis causing medicine is a diuretic. In another embodiment, a pancreatitis causing medicine is furosemide. In another embodiment, a pancreatitis causing medicine is hydrochlorothiazide. In another embodiment, a pancreatitis causing medicine is an anticonvulsant. In another embodiment, a pancreatitis causing medicine is divalproex sodium. In another embodiment, a pancreatitis causing medicine is valproic acid. In another embodiment, a pancreatitis causing medicine is L-asparaginase. In another embodiment, a pancreatitis causing medicine is azathioprine. In another embodiment, a pancreatitis causing medicine is estrogen. In another embodiment, a pancreatitis causing medicine is estrogen.

In another embodiment, the disclosure provides a method of preventing iatrogenic procedure-related acute pancreatitis. In another embodiment, the disclosure provides a method of preventing pancreatitis caused by any pancreatic surgical procedure known to one of skill in the art. In another embodiment, the disclosure provides a method of preventing pancreatitis by inhibiting necrosis. In another embodiment, the disclosure provides a method of preventing iatrogenic procedure-related acute pancreatitis comprising the step of intraperitonealy administering AAT.

In another embodiment, the disclosure provides that AAT is administered prior to the surgical procedure, during the surgical procedure, and/or after the surgical procedure. In another embodiment, the disclosure provides that AAT is administered prior to treatment with pancreatitis causing medicine, during treatment with pancreatitis causing medicine, and/or after treatment with pancreatitis causing medicine. In another embodiment, the disclosure provides that AAT is administered up to 24 hours prior to the surgical procedure or treatment with pancreatitis causing medicine. In another embodiment, the disclosure provides that AAT is administered up to 5 hours prior to the surgical procedure or treatment with pancreatitis causing medicine. In another embodiment, the disclosure provides that AAT is administered up to 15 hours prior to the surgical procedure or treatment with pancreatitis causing medicine. In another embodiment, the disclosure provides that AAT is administered up to 10 days prior to the surgical procedure or treatment with pancreatitis causing medicine. In another embodiment, the disclosure provides that AAT is administered up to 5 days prior to the surgical procedure or treatment with pancreatitis causing medicine. In another embodiment, the disclosure provides that AAT is administered up to 3 days prior to the surgical procedure or treatment with pancreatitis causing medicine.

In another embodiment, the disclosure provides that AAT is administered up to 24 hours prior to the surgical procedure or treatment with pancreatitis causing medicine. In another embodiment, the disclosure provides that AAT is administered 1-5 hours prior to the surgical procedure or treatment with pancreatitis causing medicine. In another embodiment, the disclosure provides that AAT is administered 5-15 hours prior to the surgical procedure or treatment with pancreatitis causing inedicine. In another embodiment, the disclosure provides that AAT is administered 1-10 days prior to the surgical procedure or treatment with pancreatitis causing medicine. In another embodiment, the disclosure provides that AAT is administered 1-5 days prior to the surgical procedure or treatment with pancreatitis causing medicine. In another embodiment, the disclosure provides that AAT is administered 1-3 days prior to the surgical procedure or treatment with pancreatitis causing medicine.

Alpha 1-Antitrypsin or α₁-antitrypsin (AAT, A1AT) is a glycoprotein and generally known as scrum trypsin inhibitor, may also be referred to as alpha-1 proteinase inhibitor (AIPI). AIPI is a serine protease inhibitor (serpin) inhibiting a wide variety of proteases. It protects tissues from enzymes of inflammatory cells, especially elastase, and is present in human blood at 1.5 - 3.5 gram/liter, but the concentration may increase precipitously upon acute inflammation. In its absence, as in cases of genetic deficiency, elastase is free to break down elastin, which contributes to the elasticity of the lungs resulting in respiratory complications such as emphysema leading finally to COPD (chronic obstructive pulmonary disease).

Most serpins inactivate enzymes by binding to them covalently, requiring very high levels to perform their function. In the acute phase reaction, a further elevation is required to "limit" the damage caused by activated neutrophil granulocytes and their enzyme elastase, which breaks down the connective tissue fiber elastin. Disorders of the enzyme include alpha 1-antitrypsin deficiency, a hereditary disorder in which lack of alpha I-antitrypsin leads to a chronic uninhibited tissue breakdown. This causes the subsequent degradation, especially of lung tissue and to the manifestation of pulmonary emphysema. The protein was called "antitrypsin" because of its ability to covalently bind and irreversibly inactivate the enzyme trypsin *in vitro*. Trypsin, a type of peptidase, is a digestive enzyme active in the duodenum and elsewhere. Recombinant alpha 1-antitrypsin forms are known and their use is to be considered as part of this invention. Therapeutic concentrates are prepared from the blood plasma of blood donors. Similarly, alpha-1-antitrypsin products derived from human plasma, for example, Prolactin, Zemaira and Aralast are envisioned for use according to this invention. Often such products are administered intravenously at a dose of 60 mg/kg once a week at the infusion rate of 0.08 mL/kg/min. Aerosolized augmented AAT therapy is also envisioned.

AAT inhibits a wide variety of proteases including trypsin and elastase, which are activated during the process of necrosis, resulting in a necrotic process. In some embodiments the range of effective treatments with AAT is between about 20 to 500 mg/kg/day of body weight. While in some embodiments treatment of necrosis with ATT may be conducted by intravenous injection of therapeutically effective amount of AAT, in other embodiments, treatment may comprise other administration routes, such as parenteral, oral, vaginal, rectal, nasal, buccal, intramuscular, subcutaneous, intrathecal, epidural, transdermal, intraccrebroventricular or combinations thereof. In some embodiments, administration of AAT may include an applicable carrier to allow the distribution of AAT in the blood stream or to sustain a gradual release of AAT from the injection site.

In another embodiment, effective amount of alpha-1-antitrypsin is between about 20 to 500 mg/kg/day of body weight. In another embodiment, effective amount of alpha-1-antitrypsin is between about 20 to 60 mg/kg/day of body weight. In another embodiment, effective amount of alpha-1-antitrypsin is between about 30 to 80 mg/kg/day of body weight. In another embodiment, effective amount of alpha-1-antitrypsin is between about 50 to 100 mg/kg/day of body weight. In another embodiment, effective amount of alpha-1-antitrypsin is between about 75 to 150 mg/kg/day of body weight. In another embodiment, effective amount of alpha-1-antitrypsin is between about 100 to 200 mg/kg/day of body weight. In another embodiment, effective amount of alpha-1-antitrypsin is between about 150 to 300 mg/kg/day of body weight. In another embodiment, effective amount of alpha-1-antitrypsin is between about 200 to 400 mg/kg/day of body weight. In another embodiment, effective amount of alpha-1-antitrypsin is between about 250 to 500 mg/kg/day of body weight.

In some embodiments treatment with AAT is followed by analysis of the necrotic process and determination whether the necrotic process is inhibited by the AAT treatment. This may be conducted, in some embodiments, by taking a biopsy from the site of necrosis and analysis of the biopsy with the common distinctive procedures for detection of necrosis. These assays include, but are not limited, in some embodiments, to differential staining such as the combined stain of acridine orange and ethydium bromide. Acridine orange (AO) permeates all cells and makes the nuclei appear green. Ethidium bromide (EB) is only taken up by cells when cytoplasmic membrane integrity is lost, and stains the nucleus red. EB also dominates over AO. Thus live cells have a normal green nucleus; early apoptotic cells have bright green nucleus with condensed or fragmented chromatin; late apoptotic cells display condensed and fragmented orange chromatin; cells that have died from direct necrosis have a structurally normal orange nucleus. In another embodiment, a method for measuring cytotoxicity in cells such as lactate dehydrogenase (LDH) release from dying necrotic cells can indicate necrosis. Lactate dehydrogenase is a cytosolic enzyme present within all mammalian cells. The normal plasma membrane is impermeable to LDH, but damage to the cell membrane results in a change in the membrane permeability and subsequent leakage of LDH into the extracellular fluid. In-Vitro release of LDH from cells provides an accurate measure of cell membrane integrity and cell viability. This assay is based upon the ability of LDH to catalyze the reaction: Lactate(+) + NAD(+) --> Pyruvate + NADH. Changes in optical absorbance, measured at 340nm, reflect changes in the concentration of NADH and hence the level of LDH in the test sample.

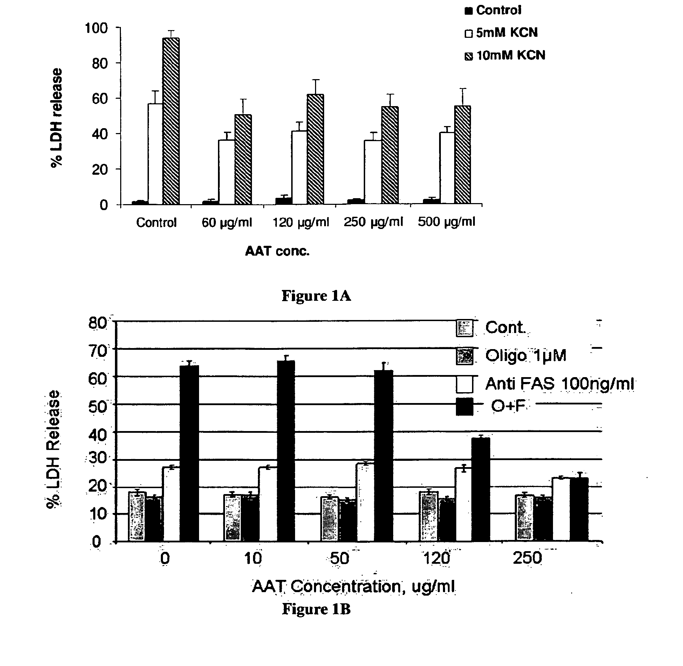

In some embodiments, cell viability assays such as trypan blue staining can be used to assess cellular necrosis. Since cells are highly selective in the compounds that pass through the membrane, in a viable cell trypan blue is not absorbed, however, it traverses the membrane in a dead cell. Hence, dead cells exhibit a distinctive blue color under a microscope. In some embodiments, treatment with AAT is followed by monitoring the availability of AAT at the necrotic tissue by taking a biopsy from the necrotic area and immunoassaying for the presence of AAT in the sample. In another embodiment, monitoring of AAT may be accomplished by imaging of AAT distribution at the site of necrosis. This can be done by linking AAT to a specific marker which enables tracking and detection using an imaging device. In some embodiments, the usage of PET scan can revel the existence of a necrotic tissue and asses the efficacy of treatment with AAT. Examples for the inhibitory effect of AAT are exemplified in Figures 1-3 herein, which show inhibition of necrosis as determined by LDH release measurement, trypan blue exclusion and ethydium bromide and acridine orange staining.

In some embodiments, treatment of necrosis may require additional medicaments to be administered in parallel to AAT. For example, in one embodiment, treatment of diabetes complications resulting in diabetic necrotic wounds may consist, in parallel to AAT, of antibiotics, anti-inflammatory drugs and insulin.

It is specifically aimed in this invention to meet the need to treat necrosis in a subject refractory to anti-inflammatory drugs. Most necrotic diseases are a result of severe inflammation leading to the development of necrotic tissue. Treatment of such diseases is done by anti-inflammatory drugs such as steroidal drugs (cortisone) and non-steroidal anti-inflammatory drug (NSAID) such as profens. In a case where the patient is non responsive to such anti-inflammatory treatment, specific treatment of the necrosis maybe an alternative solution. In some embodiments, such cases are for example cancer, neurodegenerative disease, myocardial infarction, stroke, sepsis, ischemia, liver disease, open wounds, organ transplants or gangrene. In some embodiments, the patient is immunocompromised. In one embodiment, a patient suffering from AIDS dementia, a necrotic process in brain cells specifically macrophages and microglia, may benefit from AAT treatment. Brain cells infected with HIV, secrete neurotoxins of both host and viral origin resulting in death of brain cells. The essential features of AIDS dementia are disabling cognitive impairment accompanied by motor dysfunction, speech problems and behavioral change. In one embodiment, treatment with AAT may reduce the necrotic cell death which leads to the devastating development of AIDS dementia.

This disclosure provides in some embodiments, a method of prophylactic treatment of a subject at risk for a pathological condition that is precipitated at least in part by tissue necrosis. Such conditions are, but not limited to, diabetes, cancer, neurodegenerative disease, myocardial infarction, stroke, sepsis, ischemia, liver disease and transplant patients. In such cases it might be reasonable to pre-treat with AAT to avoid the development of necrosis during the progress of the disease or due to the treatment of the disease, in the absence of effective therapy (transplantation is already covered by patent with alpha-1-antitrypsin). In some embodiments, prophylactic treatment includes administering a subject a therapeutically effective amount of alpha-1-antitrypsin to effectively inhibit the potential development of necrosis, Several biological venoms cause a rapid process of necrosis. Among these are the venom of the brown recluse spider and the rattlesnake. In one embodiment, administration of AAT to a victim of a poisonous bite may be beneficial in inhibition of the necrotic process. This may be done, in some embodiments, by injection or by topical application of AAT.

In some embodiments, the methods/compositions of this disclosure are useful in the treatment of any disease characterized by necrosis. In some embodiments, such diseases may comprise neurodegenerative disorders, leukemias, lymphmas, neonatal respiratory distress, asphyxia, incarcerated hernia, diabetes, tuberculosis, endometnosis, vascular dystrophy, psoriasis, cold injury, iron-load complications, complications of steroid treatment, ischemic heart disease, reperfusion injury, cerebrovascular disease or damage, gangrene, pressure sores, pancreatitis, hepatitis, hemoglobinuria, bacterial sepsis, viral sepsis, bums, hyperthermia, Crohn's disease, celiac disease, compartment syndrome, necrotizing procolitis, cystic fibrosis, rheumatoid arthritis, nephrotoxicity, multiple sclerosis, spinal cord injury, glomerulonephritis, muscular dystrophy, degenerative arthritis, tyromesia, metabolic inherited disease, mycoplasmal disease, anthrax inflection, bacterial infection, viral infection, Anderson disease, congenital mitochondrial disease, phenylketonuria, placental infarct, syphilis, asceptic necrosis, avascular necrosis, alcoholism and necrosis associated with administration and/or self-administration with, and/or exposure to, cocaine, drugs (e.g. paracetamol, antibiotics, adriamycin, NSAID, cydosporine) chemical toxins such as carbon tetrachloride, cyanide, methanol, ethylene glycol and mustard gas, agrochemicals such as organophosphates and aging.

Necrosis in cells or tissue culture due to lack of oxygen, inhibition of biochemical respiratory cycle, or various toxins may result in loss of the culture and the valuable time and effort invested in establishing this culture. In one embodiments, treating a culture with AAT to inhibit necrosis may lead to prevention of the loss of the culture. In another embodiment, a culture prone to necrotic cell death might serve as an experimental system for the study of necrosis. In one embodiment, supplying to such culture sufficient amount of AAT to inhibit the necrotic death and subsequent removal of ATT when assaying for the process of necrosis may result in an efficient inducible cell system for the study of necrosis. The process of necrosis is problematic in sustaining tissues and whole organs before transplantation. In one embodiment, a tissue whether a part of or a whole organ may be treated with AAT to inhibit necrosis and sustain the initial condition of the organ, or in some embodiments, allow for prolonged organ culture.

In some embodiments, "AAT" as used herein encompasses native AAT (either degradation products, synthetically synthesized AAT or recombinant AAT) and peptidomimetics (typically, synthetically synthesized polypeptides), as well as peptoids and semipeptoids which are polypeptide analogs, which have, in some embodiments, modifications rendering the polypeptides even more stable while in a body or more capable of penetrating into cells.

In another embodiment, AAT comprises the following amino acid sequence: EDPQGDAAQKTDTSHHDQDHPTFNKLTPNLAEFAFSLYRQLAHQSNSTNIFFSPVSIATAF AMLSLGTKADTHDEILEGLNFNLTEIPEAQIHEGFQELLRTLNQPDSQLQLTTGNGLFLSE GLKLVDKFLEDVKKLYHSEAFTVNFGDTEEAKKQINDYVEKGTQGKIVDLVKELDRDT VFALVNYIFFKGKWERPFEVKDTEEEDFHVDQVTTVKVPMMKRLGMFNIQHCKKLSSW VLLMKYLGNATAIFFLPDEGKLQHLENELTHDIITKFLENEDRRSASLHLPKLSITGTYDL KSVLGQLGITKVFSNGADLSGVTEEAPLSKAVHKAVLTIDEKGTEAAGAMFLEAIPM SIPPEVKFNKPFVFLMIEQNTKSPLFMGKVVNPTQK (SEQ ID NO: 1).

In another embodiment, AAT comprises peptides have AAT-like activity. In another embodiment, AAT comprises peptidyl derivatives, e.g., aldehyde or ketone derivatives of such peptides are also contemplated herein. In another embodiments, synthetic and/or naturally occurring peptides are used herein.

In another embodiment, compositions of the disclosure are used to ameliorate, reverse, and/or treat diseases and/or symptoms associated with necrosis. In another embodiment, the composition is Aralast^{™}, Baxter.

In some embodiments, modifications include, but are not limited to N-terminus modification, C terminus modification, polypeptide bond modification, including, but not limited to, CH2-NH, CH2-S, CH2-S=O, O=C-NH, CH2-O CH2-CH2, S=C-NH, CH=CH or CP=CH, backbone modifications, and residue modification, Methods for preparing peptidomimetic compounds are Well known in the art and are specified, for example, in Quantitative Drug Design, C.A. Ramsden Gd., Chapter 17.2, F. Choplin Pergamon Press (1992), which is incorporated by reference as if fully set forth herein. Further details in this respect are provided hereinunder.

In some embodiments, polypeptide bonds (-CO-NH-) within an AAT are substituted. In some embodiments, AAT bonds are substituted by N-methylated bonds (-N(CH3)-CO-). In some embodiments, the AAT bonds are substituted by ester bonds (-C(R)H-C-O-O-C(R)-N-). In some embodiments, the AAT bonds are substituted by ketomethylen bonds (-CO-CH2-). In some embodiments, the AAT bonds are substituted by α-aza bonds (-NH-N(R)-CO-), wherein R is any alkyl, e.g., methyl, carba bonds (-CH2-NH-). In some embodiments, the AAT or peptide bonds are substituted by hydroxyethylene bonds (-CH(OH)-CH2-). In some embodiments, the peptide bonds are substituted by thioamide bonds (-CS-NH-). In some embodiments, the peptide bonds are substituted by olefinic double bonds (-CH=CH-). In some embodiments, the peptide bonds are substituted by retro amide bonds (-NH-CO-). In some embodiments, the peptide bonds are substituted by peptide derivatives (-N(R)-CH2-CO-), wherein R is the "normal" side chain, naturally presented on the carbon atom. In some embodiments, these modifications occur at any of the bonds along the peptide chain and even at several (2-3 bonds) at the same time.

In some embodiments, natural aromatic amino acids of an AAT such as Trp, Tyr and Phe, are substituted for synthetic non-natural acid such as Phenylglycine, TIC, naphthylelanine (Nol), ring-methylated derivatives of Phe, halogenated derivatives of Phe or o-methyl-Tyr. In some embodiments, the AAT for use of the present invention include one or more modified amino acid or one or more non-amino acid monomers (e.g, fatty acid, complex carbohydrates etc).

In one embodiment, "amino acid" or amino acid" is understood to include the 20 naturally occurring amino acid; those amino acid often modified post-translationally *in vivo,* including, for example, hydroxyproline, phosphoserine and phosphothreonine; and other unusual amino acid including, but not limited to, 2-aminoadipic acid, hydroxylysine, isodesmosine, nor-valine, nor-leucine and ornithine. In one embodiment, "amino acid" includes both D- and L-amino acid.

In some embodiments, the AAT for use of the present invention are utilized in therapeutics which requires the AAT to be in a soluble form. In some embodiments, the AAT for use of the present invention include one or more non-natural or natural polar amino acid, including but not limited to serine and threonine which are capable of increasing polypeptide solubility due to their hydroxyl-containing side chain.

In some embodiments, the AAT for use of the present invention is utilized in a linear form, although it will be appreciated by one skilled in the art that in cases where cyclicization does not severely interfere with AAT characteristics, cyclic forms of the AAT can also be utilized.

In some embodiments the AAT for use of present invention is biochemically synthesized such as by using standard solid phase techniques. In some embodiments, these biochemical methods include exclusive solid phase synthesis, partial solid phase synthesis, fragment condensation, or classical solution synthesis.

In some embodiments, solid phase AAT synthesis procedures are well known to one skilled in the art and further described by John Morrow Stewart and Janis Dillaha Young, Solid Phase Polypeptide Syntheses (2nd Ed., Pierce Chemical Company, 1984). In some embodiments, synthetic AAT is purified by preparative high performance liquid chromatography [Creighton T. (1983) Proteins, structures and molecular principles. WH Freeman and Co. N.Y.] and the composition of which can be confirmed via amino acid sequencing by methods known to one skilled in the art.

In some embodiments, recombinant protein techniques are used to generate the AAT for use of the present invention. In some embodiments, recombinant protein techniques are used for generation of an AAT (e.g., longer than 18-25 amino acids). In some embodiments, recombinant protein techniques are used for the generation of large amounts of the polypeptide of the present disclosure. In some embodiments, recombinant techniques are described by Bitter et al., (1987) Methods in Enzymol. 153:516-544, Studier et al. (1990) Methods in Enzymol. 185:60-89, Brisson et at (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VII, pp 421-463..

In one embodiment, an AAT for use of the present invention is synthesized using a polynucleotide encoding an AAT for use of the present invention. In some embodiments, the polynucleotide encoding an AAT for use of the present invention is ligated into an expression vector, comprising a transcriptional control of a cis-regulatory sequence (e.g., promoter sequence). In some embodiments, the cis-regulatory sequence is suitable for directing constitutive expression of the AAT for use of the present invention.

In some embodiments, the cis-regulatory sequence is suitable for directing tissue specific expression of the AAT for use of the present invention. In some embodiments, the cis-regulatory sequence is suitable for directing inducible expression of the AAT for use of the present invention.

In some embodiment, tissue-specific promoters suitable for use with the present invention include sequences which are functional in specific cell population, example include, but are not limited to promoters such as albumin that is liver specific [Pinkert et al., (1987) Genes Dev. 1:268-277], lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byrne et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166). Inducible promoters suitable for use with the present invention include for example the tetracycline-inducible promoter (Srour, M.A., et al., 2003. Thromb. Haemost. 90: 398-405).

In one embodiment, the phrase "a polynucleotide" refers to a single or double stranded nucleic acid sequence which be isolated and provided in the form of an RNA sequence, a complementary polynucleotide sequence (cDNA), a genomic polynucleotide sequence and/or a composite polynucleotide sequences (e.g., a combination of the above).

In one embodiment, "complementary polynucleotide sequence" refers to a sequence, which results from reverse transcription of messenger RNA using a reverse transcriptase or any other RNA dependent DNA polymerase. In one embodiment, the sequence can be subsequently amplified *in vivo* or *in vitro* using a DNA polymerase.

In one embodiment, "genomic polynucleotide sequence" refers to a sequence derived (isolated) from a chromosome and thus it represents a contiguous portion of a chromosome.

In one embodiment, "composite polynucleotide sequence" refers to a sequence, which is at least partially complementary and at least partially genomic. In one embodiment, a composite sequence can include some exonal sequences required to encode the polypeptide of the present disclosure, as well as some intronic sequences interposing therebetween. In one embodiment, the intronic sequences can be of any source, including of other genes, and typically will include conserved splicing signal sequences. In one embodiment, intronic sequences include cis acting expression regulatory elements.

In some embodiments, polynucleotides of the present disclosure are prepared using PCR techniques, or any other method or procedure known to one skilled in the art. In some embodiments, the procedure involves the ligation of two different DNA sequences (See, for example, "Current Protocols in Molecular Biology", eds. Ausubel et al., John Wiley & Sons. 1992).

In one embodiment, polynucleotides of the present disclosure are inserted into expression vectors (i.e., a nucleic acid construct) to enable expression of the recombinant polypeptide. In one embodiment, the expression vector of the present disclosure includes additional sequences which render this vector suitable for replication and integration in prokaryotes. In one embodiment, the expression vector of the present disclosure includes additional sequences which render this vector suitable for replication and integration in eukaryotes. In one embodiment, the expression vector of the present disclosure includes a shuttle vector which renders this vector suitable for replication and integration in both prokaryotes and eukaryotes. In some embodiments, cloning vectors comprise transcription and translation initiation sequences (e.g., promoters, enhances) and transcription and translation terminators (e.g., polyadenylation signals).

In one embodiment, a variety of prokaryotic or eukaryotic cells can be used as host-expression systems to express the polypeptides of the present disclosure. In some embodiments, these include, but are not limited to, microorganism, such as bacteria transformed with a recombinant bacteriophage DNA, plasmid DNA or cosmid DNA expression vector containing the polypeptide coding sequence; yeast transformed with recombinant yeast expression vectors containing the polypeptide coding sequence; plant cell systems infected with recombinant virus expression vectors (e.g., cauliflower mosaic virus, CaMV; tobacco mosaic vims, TMV) or transformed with recombinant plasmid expression vectors, such as Ti plasmid, containing the polypeptide coding sequence.

In some embodiments, non-bacterial expression systems are used (e.g. mammalian expression systems such as CHO cells) to express the polypeptide of the present disclosure. In one embodiment, the expression vector used to express polynucleotides of the present disclosure in mammalian cells is pCI-DHFR vector comprising a CMV promoter and a neomycin resistance gene.

In some embodiments, in bacterial systems of the present disclosure, a number of expression vectors can be advantageously selected depending upon the use intended for the AAT expressed. In one embodiment, large quantities of AAT arc desired. In one embodiment, vectors that direct the expression of high levels of the AAT protein product, possibly as a fusion with a hydrophobic signal sequence, which directs the expressed product into the periplasm of the bacteria or the culture medium where the protein product is readily purified are desired. In one embodiment, certain fusion protein engineered with a specific cleavage site to aid in recovery of the polypeptide. In one embodiment, vectors adaptable to such manipulation include, but are not limited to, the pET series of *E*. *coli* expression vectors [Studier et al., Methods in Enzymol. 185:60-89 (1990)].

In one embodiment, yeast expression systems are used. In one embodiment, a number of vectors containing constitutive or inducible promoters can be used in yeast as disclosed in U.S. Pat. Application No: 5,932,447. In another embodiment, vectors which promote integration of foreign DNA sequences into the yeast chromosome are used.

In one embodiment, the expression vector of the present disclosure can further include additional polynucleotide sequences that allow, for example, the translation of several proteins from a single mRNA such as an internal ribosome entry site (IRES) and sequences for genomic integration of the promoter-chimeric AAT.

In some embodiments, mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

In some embodiments, expression vectors containing regulatory elements from eukaryotic viruses such as retroviruses are used by the present invention. SV40 vectors include pSVT7 and pMT2. In some embodiments, vectors derived from bovine papilloma virus include pBV-1MTHA, and vectors derived from Epstein Bar virus include pHEBO, and p2O5 Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

In some embodiments, recombinant viral vectors are useful for *in vivo* expression of the AAT of the present disclosure since they offer advantages such as lateral infection and targeting specificity. In one embodiment, lateral infection is inherent in the life cycle of, for example, retrovirus and is the process by which a single infected cell produces many progeny virions that bud off and infect neighboring cells. In one embodiment, the result is that a large area becomes rapidly infected, most of which was not initially infected by the original viral particles. In one embodiment, viral vectors are produced that are unable to spread laterally. In one embodiment, this characteristic can be useful if the desired purpose is to introduce a specified gene into only a localized number of targeted cells.

In one embodiment, various methods can be used to introduce the expression vector of the present disclosure into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich, (1995), Vectors: A Survey of Molecular Cloning Vectors and Their Uses, Butterworths, Boston Mass. (1988) and Gilboa et at [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

In some embodiments, introduction of nucleic acid by viral infection offers several advantages over other methods such as lipofection and electroporation, since higher transfection efficiency can be obtained due to the infectious nature of viruses.

In one embodiment, it will be appreciated that the AAT for use of the present invention can also be expressed from a nucleic acid construct administered to the individual employing any suitable mode or administration, described hereinabove (i.e., in-vivo gene therapy). In one embodiment, the nucleic acid construct is introduced into a suitable cell via an appropriate gene delivery vehicle/method (transfection, transduction, homologous recombination, etc.) and an expression system as needed and then the modified cells are expanded in culture and returned to the individual (i.e., ex-vivo gene therapy).

In one embodiment, plant expression vectors are used. In one embodiment, the expression of a polypeptide coding sequence is driven by a number of promoters. In some embodiments, viral promoters such as the 35S RNA and 19S RNA promoters of CaMV [Brisson et al., Nature 310:511-514 (1984)], or the coat protein promoter to TMV [Takamatsu et al., EMBO J. 6:307-311 (1987)] are used. In another embodiment,, plant promoters are used such as, for example, the small subunit of RUBISCO [Coruzzi et al., EMBO J. 3:1671-1680 (1984); and Brogli et al., Science 224:838-843 (1984)] or heat shock promoters, e,g., soybean hsp17.5-E or hsp17.3-H [Gurley et al., Mol. Cell. Biol. 6:559-565 (1986)]. In one embodiment, constructs are introduced into plant cells using Ti plasmid, Ri plasmid, plant viral vectors, direct DNA transformation, microinjection, electroporation and other techniques well known to the skilled artisan. See, for example, Weissbach & Weissbach [Methods for Plant Molecular Biology, Academic Press, NY, Section VIII, pp 421-463 (1988)]. Other expression systems such as insects and mammalian host cell systems, which are well known in the art, can also be used by the present invention.

It will be appreciated that other than containing the necessary elements for the transcription and translation of the inserted coding sequence (encoding the AAT), the expression construct of the present disclosure can also include sequences engineered to optimize stability, production, purification, yield or activity of the expressed AAT.

Various methods, in some embodiments, can be used to introduce the expression vector of the present disclosure into the host cell system. In some embodiments, such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

In some embodiments, transformed cells are cultured under effective conditions, which allow for the expression of high amounts of recombinant AAT. In some embodiments, effective culture conditions include, but are not limited to, effective media, bioreactor, temperature, pH and oxygen conditions that permit protein production. In one embodiment, an effective medium refers to any medium in which a cell is cultured to produce the recombinant polypeptide of the present invention. In some embodiments, a medium typically includes an aqueous solution having assimilable carbon, nitrogen and phosphate sources, and appropriate salts, minerals, metals and other nutrients, such as vitamins. In some embodiments, cells of the present invention can be cultured in conventional fermentation bioreactors, shake flasks, test tubes, microliter dishes and petri plates. In some embodiments, culturing is carried out at a temperature, pH and oxygen content appropriate for a recombinant cell. In some embodiments, culturing condition are within the expertise of one of ordinary skill in the art:

In some embodiments, depending on the vector and host system used for production, resultant AAT for use of the present invention either remain within the recombinant cell, secreted into the fermentation medium, secreted into a space between two cellular membranes, such as the periplasmic space in *E. coli*; or retained on the outer surface of a cell or viral membrane.

In one embodiment, following a predetermined time in culture, recovery of the recombinant AAT is effected.

In one embodiment, the phrase "recovering the recombinant AAT" used herein refers to collecting the whole fermentation medium containing the AAT and need not imply additional steps of separation or purification,

In one embodiment, AAT for use of the present invention is purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization.

In one embodiment, to facilitate recovery, the expressed coding sequence can be engineered to encode the AAT for use of the present invention and fused cleavable moiety. In one embodiments, a fusion protein can be designed so that the AAT can be readily isolated by affinity chromatography; e.g., by immobilization on a column specific for the cleavable moiety. In one embodiment, a cleavage site is engineered between the polypeptide and the cleavable moiety and the polypeptide can be released from the chromatographic column by treatment with an appropriate enzyme or agent that specifically cleaves the fusion protein at this site (e.g., see Booth et al., Immunol. Lett. 19:65-70 (1988); and Gardella et al., J. BioL Chem. 265:15854-15859 (1990)].

In one embodiment, the AAT for use of the present invention is retrieved in "substantially pure" form.

In one embodiment, the phrase "substantially pure" refers to a purity that allows for the effective use of the protein in the applications described herein.

In one embodiment, the AAT for use of the present invention can also be synthesized using *in vitro* expression systems. In one embodiment, *in vitro* synthesis methods are well known in the art and the components of the system are commercially available.

In one embodiment, a "pharmaceutical composition" refers to a preparation of one or more of the active ingredients described herein with other chemical components such as physiologically suitable carriers and excipients. The purpose of a pharmaceutical composition is to facilitate administration of an AAT to an organism.

In one embodiment, "active ingredient" refers to the AAT sequence of interest, which is accountable for the biological effect. In one embodiment, "active ingredient" refers to the AAT protein, which is accountable for the biological effect.

In some embodiments, any of the compositions of this disclosure will comprise at least ATT in any form. In one embodiment, the present disclosure provides combined preparations. In one embodiment, "a combined preparation" defines especially a "kit of parts" in the sense that the combination partners as defined above can be dosed independently or by use of different fixed combinations with distinguished amounts of the combination partners i.e., simultaneously, concurrently, separately or sequentially. In some embodiments, the parts of the kit of parts can then, e.g., be administered simultaneously or chronologically staggered, that is at different time points and with equal or different time intervals for any part of the kit of parts. The ratio of the total amounts of the combination partners, in some embodiments, can be administered in the combined preparation. In one embodiment, the combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

In one embodiment, the phrases "physiologically acceptable carrier" and "pharmaceutically acceptable carrier" which be interchangeably used refer to a carrier or a diluent that does not cause significant irritation to an organism and does not abrogate the biological activity and properties of the administered compound. An adjuvant is included under these phrases. In one embodiment, one of the ingredients included in the pharmaceutically acceptable carrier can be for example polyethylene glycol (PEG), a biocompatible polymer with a wide range of solubility in both organic and aqueous media (Mutter et al. (1979).

In one embodiment, "excipient" refers to an inert substance added to a pharmaceutical composition to further facilitate administration of an active ingredient. In one embodiment, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition, which is incorporated herein by reference.

In one embodiment, suitable routes of administration, for example, include oral, rectal, transmucosal, transnasal, intestinal or parenteral delivery, including intramuscular, subcutaneous and intramedullary injections as well as intrathecal, direct intraventricular, intravenous, inrtaperitoneal, intranasal, or intraocular injections.

In one embodiment, the preparation is administered in a local rather than systemic manner, for example, via injection of the preparation directly into a specific region of a patient's body.

Various embodiments of dosage ranges are contemplated by this invention. The dosage of the AAT for use of the present invention, in one embodiment, is in the range of 0.05-80 mg/day. In another embodiment, the dosage is in the range of 0.05-50 mg/day. In another embodiment, the dosage is in the range of 0.1-20 mg/day. In another embodiment, the dosage is in the range of 0.1-10 mg/day. In another embodiment, the dosage is in the range of 0.1-5 mg/day. In another embodiment, the dosage is in the range of 0.5-5 mg/day. In another embodiment, the dosage is in the range of 0.5-50 mg/day. In another embodiment, the dosage is in the range of 5-80 mg/day. In another embodiment, the dosage is in the range of 35-65 mg/day. In another embodiment, the dosage is in the range of 35-65 mg/day. In another embodiment, the dosage is in the range of 20-60 mg/day. In another embodiment, the dosage is in the range of 40-60 mg/day. In another embodiment, the dosage is in a range of 45-60 mg/day. In another embodiment, the dosage is in the range of 40-60 mg/day. In another embodiment, the dosage is in a range of 60-120 mg/day. In another embodiment, the dosage is in the range of 120-240 mg/day. In another embodiment, the dosage is in the range of 40-60 mg/day. In another embodiment, the dosage is in a range of 240-500 mg/day. In another embodiment, the dosage is in a range of 45-60 mg/day. In another embodiment, the dosage is in the range of 15-25 mg/day. In another embodiment, the dosage is in the range of 5-10 mg/day. In another embodiment, the dosage is in the range of 55-65 mg/day.

In one embodiment, the dosage is 20 mg/day. In another embodiment, the dosage is 30 mg/day. In another embodiment, the dosage is 40 mg/day. In another embodiment, the dosage is 50 mg/day. In another embodiment, the dosage is 60 mg/day. In another embodiment, the dosage is 70 mg/day. In another embodiment, the dosage is 80 mg/day. In another embodiment, the dosage is 90 mg/day. In another embodiment, the dosage is 100 mg/day.

Oral administration, in one embodiment, comprises a unit dosage form comprising tablets, capsules, lozenges, chewable tablets, suspensions, emulsions and the like. Such unit dosage forms comprise a safe and effective amount of the desired compound, or compounds, each of which is in one embodiment, from about 0.7 or 3.5 mg to about 280 mg/70 kg, or in another embodiment, about 0.5 or 10 mg to about 210 mg/70 kg. The pharmaceutically-acceptable carriers suitable for the preparation of unit dosage forms for peroral administration are well-known in the art. In some embodiments, tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. In one embodiment, glidants such as silicon dioxide can be used to improve flow characteristics of the powder-mixture. In one embodiment, coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. In some embodiments, the selection of carrier components depends on secondary considerations like taste, cost, and shelf stability, which are not critical for the purposes of this invention, and can be readily made by a person skilled in the art.

In one embodiment, the oral dosage form comprises predefined release profile. In one embodiment, the oral dosage form of the present disclosure comprises an extended release tablets, capsules, lozenges or chewable tablets. In one embodiment, the oral dosage form of the present disclosure comprises a slow release tablets, capsules, lozenges or chewable tablets. In one embodiment, the oral dosage form of the present disclosure comprises an Immediate release tablets, capsules, lozenges or chewable tablets. In one embodiment, the oral dosage form is formulated according to the desired release profile of the pharmaceutical active ingredient as known to one skilled in the art.

Peroral compositions, in some embodiments, comprise liquid solutions, emulsions, suspensions, and the like. In some embodiments, pharmaceutically-acceptable carriers suitable for preparation of such compositions are well known in the art. In some embodiments, liquid oral compositions comprise from about 0.012% to about 0.933% of the desired compound or compounds, or in another embodiment, from about 0.033% to about 0.7%.

In some embodiments, compositions for use in the methods of this disclosure comprise solutions or emulsions, which in some embodiments are aqueous solutions or emulsions comprising a safe and effective amount of the compounds of the present disclosure and optionally, other compounds, intended for topical intranasal administration. In some embodiments, the compositions comprise from about 0.01% to about 10.0% w/v of a subject compound, more preferably from about 0.1% to about 2.0, which is used for systemic delivery of the compounds by the intranasal route.

In another embodiment, the pharmaceutical compositions are administered by intravenous, intraarterial, or intramuscular injection of a liquid preparation. In some embodiments, liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intra-arterially, and are thus formulated in a form suitable for intra-arterial administration. In another embodiment, the pharmaceutical compositions are administered intramuscularly, and are thus formulated in a form suitable for intramuscular administration.

Further, in another embodiment, the pharmaceutical compositions are administered topically to body surfaces, and are thus formulated in a form suitable for topical administration. Suitable topical formulations include gels, ointments, creams, lotions, drops and the like. For topical administration, the compounds of the present disclosure are combined with an additional appropriate therapeutic agent or agents, prepared and applied as solutions, suspensions, or emulsions in a physiologically acceptable diluent with or without a pharmaceutical carrier.

In one embodiment, pharmaceutical compositions of the present disclosure are manufactured by processes well known in the art, e.g., by means of conventional mixing, dissolving, granulating, dragee-making, levigating, emulsifying, encapsulating, entrapping or lyophilizing processes.

In one embodiment, pharmaceutical compositions for use in accordance with the present disclosure is formulated in conventional manner using one or more physiologically acceptable carriers comprising excipients and auxiliaries, which facilitate processing of the active ingredients into preparations which, can be used pharmaceutically. In one embodiment, formulation is dependent upon the route of administration chosen.

In one embodiment, injectables, of the disclosure are formulated in aqueous solutions. In one embodiment, injectables, of the disclosure are formulated in physiologically compatible buffers such as Hank's solution, Ringer's solution, or physiological salt buffer. In some embodiments, for transmucosal administration, penetrants appropriate to the barrier to be permeated are used in the formulation. Such penetrants are generally known in the art.

In one embodiment, the preparations described herein are formulated for parenteral administration, e.g., by bolus injection or continuous infusion. In some embodiments, formulations for injection are presented in unit dosage form, e.g., in ampoules or in multi-dose containers with optionally, an added preservative. In some embodiments, compositions are suspensions, solutions or emulsions in oily or aqueous vehicles, and contain formulatory agents such as suspending, stabilizing and/or dispersing agents.

The compositions also comprise, in some embodiments, preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjusters, such as polymers, including cellulose and derivatives thereof; and polyvinyl alcohol and acid and bases to adjust the pH of these aqueous compositions as needed. The compositions also comprise, in some embodiments, local anesthetics or other actives. The compositions can be used as sprays, mists, drops, and the like.

In some embodiments, pharmaceutical compositions for parenteral administration include aqueous solutions of the active preparation in water-soluble form. Additionally, suspensions of the active ingredients, in some embodiments, are prepared as appropriate oily or water based injection suspensions. Suitable lipophilic solvents or vehicles include, in some embodiments, fatty oils such as sesame oil, or synthetic fatty acid esters such as ethyl oleate, triglycerides or liposomes. Aqueous injection suspensions contain, in some embodiments, substances, which increase the viscosity of the suspension, such as sodium carboxymethyl cellulose, sorbitol or dextran. In another embodiment,, the suspension also contain suitable stabilizers or agents which increase the solubility of the active ingredients to allow for the preparation of highly concentrated solutions.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

In another embodiment, the pharmaceutical composition delivered in a controlled release system is formulated for intravenous infusion, implantable osmotic pump, transdermal patch, liposomes, or other modes of administration. In one embodiment, a pump is used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

In some embodiments, the active ingredient is in powder form for constitution with a suitable vehicle, e.g., sterile, pyrogen-free water based solution, before use. Compositions are formulated, in some embodiments, for atomization and inhalation administration. In another embodiment, compositions are contained in a container with attached atomizing means.

In some embodiments, pharmaceutical compositions suitable for use in context of the present disclosure include compositions wherein the AAT is contained in an amount effective to achieve the intended purpose. In some embodiments, a therapeutically effective amount means an amount of AAT effective to prevent, alleviate or ameliorate symptoms of disease associated with necrosis or prolong the survival of the subject being treated.

In one embodiment, determination of a therapeutically effective amount is well within the capability of those skilled in the art.

The compositions also comprise preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic; acid, acetylcystine, sodium metabisulfote and others; aromatic agents; viscosity adjustors, such as polymers, including cellulose and derivatives thereof; and polyvinyl alcohol and acid and bases to adjust the pH of these aqueous compositions as needed. The compositions also comprise local anesthetics or other actives. The compositions can be used as sprays, mists, drops, and the like.

Some examples of substances which can serve as pharmaceutically-acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the Tween™ brand emulsifiers; wetting agents, such sodium lauryl sulfate; coloring agents; flavoring agents; tableting agents, stabilizers; antioxidants; preservatives; pyrogen-free water; isotonic saline; and phosphate buffer solutions. The choice of a pharmaceutically-acceptable carrier to be used in conjunction with the compound is basically determined by the way the compound is to be administered. If the subject compound is to be injected, in one embodiment, the pharmaceutically-acceplable carrier is sterile, physiological saline, with a blood-compatible suspending agent, the pH of which has been adjusted to about 7.4.

In addition, the compositions further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, cellulose (e.g. Avicel™, RC-591), tragacanth and sodium alginate; typical wetting agents include lecithin and polyethylene oxide sorbitan (e.g. polysorbate 80). Typical preservatives include methyl paraben and sodium benzoate. In another embodiment, peroral liquid compositions also contain one or more components such as sweeteners, flavoring agents and colorants disclosed above.

The compositions also include incorporation of the AAT into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate of *in vivo* release, and rate of *in vivo* clearance.

Also comprehended by the disclosure are particulate compositions coated with polymers (e.g. poloxamers or poloxamines) and the compound coupled to antibodies directed against tissue-specific receptors, ligands or antigens or coupled to ligands of tissue-specific receptors.

In some embodiments, compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. In another embodiment, the modified compounds exhibit substantially longer half-lives in blood following intravenous injection than do the corresponding unmodified compounds. In one embodiment, modifications also increase the compound's solubility in aqueous solution, eliminate aggregation, enhance the physical and chemical stability of the compound, and greatly reduce the immunogenicity and reactivity of the compound. In another embodiment, the desired *in vivo* biological activity is achieved by the administration of such polymer-compound abducts less frequently or in lower doses than with the unmodified compound.

In some embodiments, preparation of effective amount or dose can be estimated initially from in vitro assays. In one embodiment, a dose can be formulated in animal models and such information can be used to more accurately determine useful doses in humans,

In one embodiment, toxicity and therapeutic efficacy of the active ingredients described herein can be determined by standard pharmaceutical procedures *in vitro*, in cell cultures or experimental animals. In one embodiment, the data obtained from these *in vitro* and cell culture assays and animal studies can be used in formulating a range of dosage for use in human. In one embodiment, the dosages vary depending upon the dosage form employed and the route of administration utilized. In one embodiment, the exact formulation, route of administration and dosage can be chosen by the individual physician in view of the patient's condition. [See e.g., Fingl, et al., (1975) "The Pharmacological Basis of Therapeutics", Ch. 1 p.l].

In one embodiment, depending on the severity and responsiveness of the condition to be treated, dosing can be of a single or a plurality of administrations, with course of treatment lasting from several days to several weeks or until cure is effected or diminution of the disease state is achieved.

In one embodiment, the amount of a composition to be administered will, of course, be dependent on the subject being treated, the severity of the affliction, the manner of administration, the judgment of the prescribing physician, etc.

In one embodiment, compositions including the preparation of the present disclosure formulated in a compatible pharmaceutical carrier are also be prepared, placed in an appropriate container, and labeled for treatment of an indicated condition.

In one embodiment, compositions of the present disclosure are presented in a pack or dispenser device, such as an FDA approved kit, which contain one or more unit dosage forms containing the active ingredient. In one embodiment, the pack, for example, comprise metal or plastic foil, such as a blister pack. In one embodiment, the pack or dispenser device is accompanied by instructions for administration. In one embodiment, the pack or dispenser is accommodated by a notice associated with the container in a form prescribed by a governmental agency regulating the manufacture, use or sale of pharmaceuticals, which notice is reflective of approval by the agency of the form of the compositions or human or veterinary administration. Such notice, in one embodiment, is labeling approved by the U.S. Food and Drug Administration for prescription drugs or of an approved product insert.

In one embodiment, it will be appreciated that the polypeptides of the present disclosure can be provided to the individual with additional active agents to achieve an improved therapeutic effect as compared to treatment with each agent by itself. In another embodiment, measures (e.g., dosing and selection of the complementary agent) are taken to adverse side effects which are associated with combination therapies.

Additional objects; advantages, and novel features of the present invention will become apparent to one ordinarily skilled in the art upon examination of the following examples, which are not intended to be limiting. Additionally, each of the various embodiments and aspects of the present disclosure as delineated hereinabove and as claimed in the claims section below finds experimental support in the following examples.

In one embodiment, the polypeptides of the present disclosure can be provided to the individual *per se*. In one embodiment, the polypeptides of the present disclosure can be provided to the individual as part of a pharmaceutical composition where it is mixed with a pharmaceutically acceptable carrier.

While the disclosure has been described, it is not intended to be limited to the details shown, since various modifications and substitutions can be made without departing in any way from the spirit of the present disclosure. As such, further modifications and equivalents of the invention herein disclosed can occur to persons skilled in the art using no more than routine experimentation.

### EXAMPLES

### MATERIAL AND EXPERIMENTAL METHODS

Cell culture. U-937 cells or PC-12 cell lines, were cultured in RPMI 1640 medium with 10% fetal calf serum, glutamine and a combination of penicillin and streptomycin. The cells were maintained at logarithmic phase. Cells were grown at 37°C in a humidified 5% CO₂ atmosphere. For the experiment, cells were maintained in glucose-free medium for 1 h, pre-incubated with or without AAT for 30 min and then KCN was added for seven hours for induction of necrosis.

**Methods for assessing tell viability.** Cell viability was assessed under light microscope by trypan blue staining - dead cells are stained blue while viable cells remain transparent. Another method employed Promega's CytoTox^{®} 96 non-radioactive cytotoxicity assay which accurately and rapidly measures cell death by quantitating the release of lactate dehydrogenase (LDH), a stable cytosolic enzyme, from lysed cells. The cell death pathway was determined by ethydium bromide and acridine orange double staining. This system allows one to distinguish between live, necrotic and apoptotic cells and also to determine if the cells are in an early or late stage of apoptosis.

### RESULTS

### EXAMPLE 1

### Effect of AAT on KCN-induced necrosis

In order to establish AAT effect on necrosis, cells were maintained in glucose-free medium, pre-incubated with or without AAT (0.5 mg/ml) for 230 min and KCN was added for seven hours to induce necrosis. LDH release was determined using Promega's CytoTox 96^{®}. Figure 1A shows that Alpha 1-anti-trypsin caused a stable decrease in the LDH release after incubation with KCN as compared to controls.

Figure 1b shows that AAT caused a stable decrease in the LDH release in PC 12 cells cells treated with oligomycin. Specifically, these cells were exposed to oligomycin 1 µM and/or 100 ng/ml anti-Pas induced cell death in the presence or in the absence of different concentrations of AAT for 18 hours and then LDH release from the cells was determined.

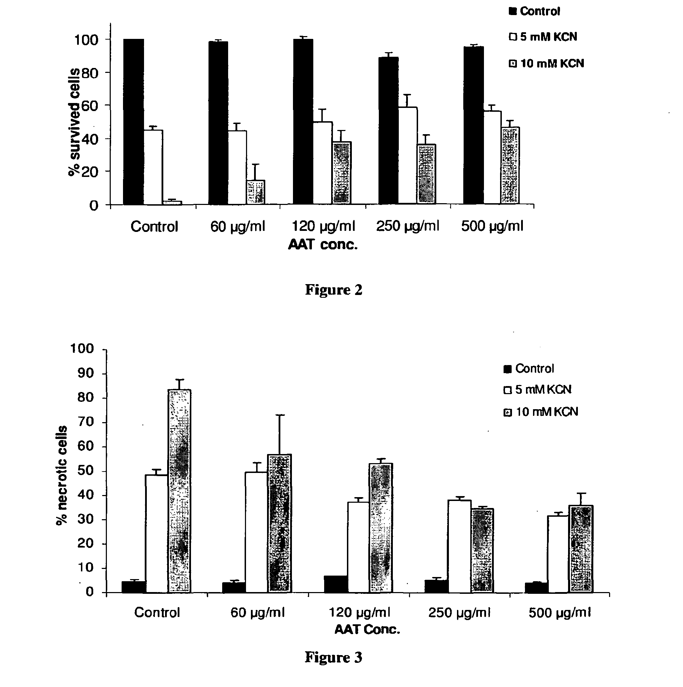

In another test of AAT effects on necrosis, cells were maintained in glucose-free medium, pre-incubated with or without AAT for 30 minutes and KCN was added for seven hours to induce necrosis. After 7 hours, live and dead cells were stained and counted by trypan blue exclusion. Figure 2 shows an increase in surviving cells as a result of AAT treatment after incubation with KCN, indicating inhibition of necrosis.

To further test the inhibitory effect of AAT on necrosis, cells were maintained in glucose-free medium, pre-incubated with or without AAT for 30 min and then KCN was added for seven hours. After 7 hours, necrotic cells were stained and counted by ethydium bromide and acridine orange double staining. Acridine orange (AO) permeates all cells and makes the nuclei appear green. Ethidium bromide (EB) is only taken up by cells when cytoplasmic membrane integrity is lost, and stains the nucleus red. EB also dominates over AO. Thus live cells have a normal green nucleus; early apoptotic cells have bright green nucleus with condensed or fragmented chromatin; late apoptotic cells display condensed and fragmented orange chromatin; cells that have died from direct necrosis have a structurally normal orange nucleus. Figure 3 depicts a decrease in the percent of necrotic cells resulting from pre-incubation with AAT for 30 min prior to incubation with KCN. These results indicate an inhibitory effect of AAT on necrosis.

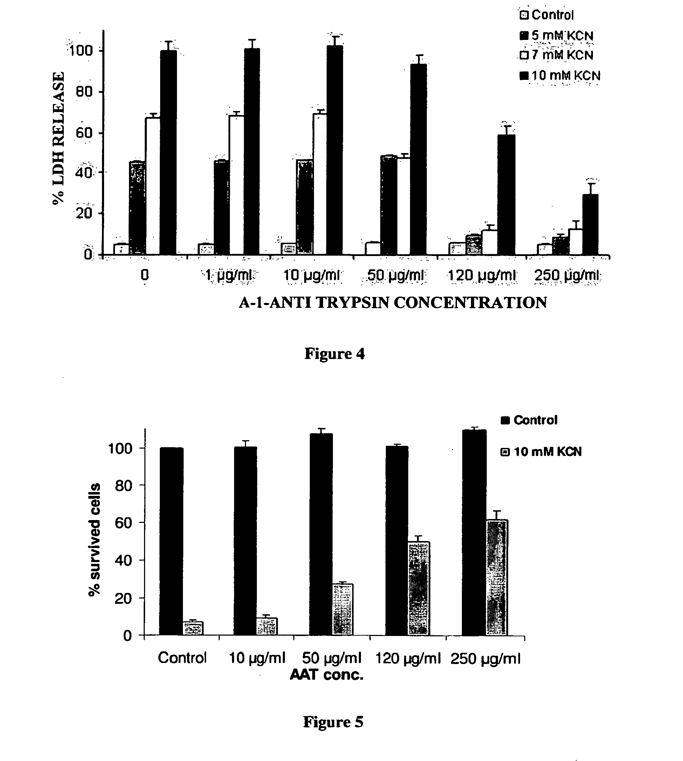

Then the protective effect of AAT on KCN-induced necrosis in PC-12 cells was assessed. The cells were maintained in glucose-free medium, pre-incubated with or without AAT for 30 min and then KCN was added for five hours (Figure 4). Thereafter LDH release was determined. The results clearly reveal that alpha-1-antitrypsin has a pronounced protective anti-necrotic activity as indicated by a stable decrease in the LDH release. Moreover, as shown in Figure 5, ATT protects PC 12 cells from necrosis in a dose dependent manner.

### EXAMPLE 2

### Preventative Alpha-1-Antitrypsin Therapy in Iatrogenic Acute Pancreatitis

Iatrogenic procedure-related acute pancreatitis results in an unacceptable high rate of morbidity and mortality. Complications might result from widely used surgical procedures such as endoscopic retrograde cholangiopancreatography (ERCP), pancreatic stenting, pancreaticoduodenectomy and pancreatectomy.

The pathogenesis of procedure-related acute pancreatitis has involves massive activation of trypsinogen, the primary protease activator of accompanying pancreatic proteolytic zymogens within the pancreatic gland. Trypsinogen activity can be blocked by a number of naturally occurring protease inhibitors that are produced by the pancreas, including alpha-1-antitrypsin (AAT), pancreatic secretory protease inhibitor and alpha-2-macroglobulin. However, in cases of pancreatic injury, these inhibitors may become saturated and their net inhibitory function inadequate to prevent extensive tissue injury.

In this study human alpha-1-antitrypsin was intraperitonealy administered as a preventative measure during abdominal surgery-related pancreatitis. Endpoints of study include animal survival, animal morbidity, and severity of pancreatitis as assessed by circulating pancreatic lipase activity.

### Materials and methods

Mice (C57B1/6, 6-7 week old females, Harlan, Israel) were anesthetized by a standard injection of ketamine/xylasine. A 1 cm long transverse abdominal incision was performed in the upper middle quadrant to expose the pancreatic duct. The duct was ligated by 3-0 sterile suture in a double-knot, and the surgical opening closed.

Treatments included SHAM operated mice (opening, no ligation, n = 3), ligated mice that were injected PBS 1 hour prior to procedure (n = 5), and AAT-treated animals, that were administered human AAT (AralastTM, Baxter, 60 mg/kg i.p., 1 hour prior to procedure, n = 7).

Follow-up consisted of recording of animal behavior on a scale of 1 to 5 (1 viable, 5 morbid). Lipase activity was assessed in sera 24 hours after ligation using standard lipase enzymatic assay (Sigma, Rehovot, Israel). Pancreata were removed at the end of the experiment for macroscopic evaluation and images obtained.

### Results

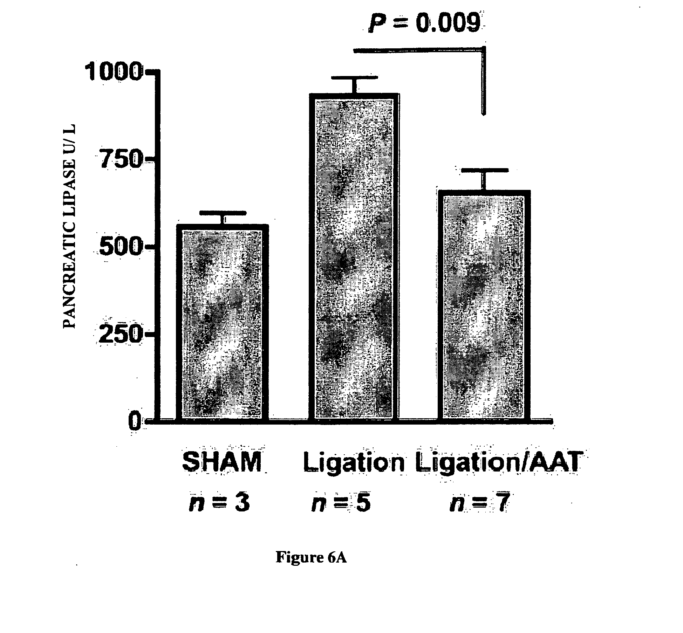

As shown, while serum pancreatic lipase was elevated after ligation (Figure 6A), animals that were treated with AAT exhibited significantly lower levels of the pancreatic enzyme, approaching healthy values.

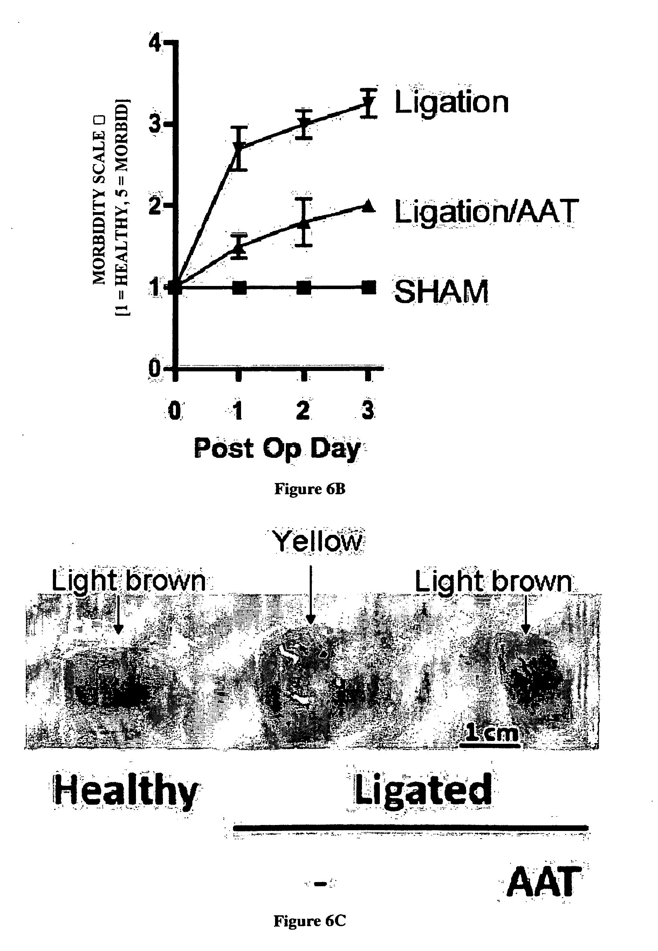

Morbidity, as assessed on a daily basis, was lower in ligated AAT-treated animals than control ligated animals (Figure 6B). At harvest abdominal investigation revealed a highly jaundiced liver in all ligated animals; however, macroscopic inspection of pancreata from AAT-treated ligated animals closely resembled healthy pancreata, while untreated animals displayed a distended, partially digested pathological pancreas (Figure 6C). The yellow discoloring as indicated in the figure (6C) of the affected pancreas is jaundice that is attributed to the hepatic injury, secondary to common bile duct ligation.

## Claims

1. Use of a therapeutically effective amount of alpha-1-antitrypsin or an active homologue or an active variant thereof for the preparation of a medicament for treatment of tissue necrosis, wherein the medicament is to be administered to a tissue **characterized by** undergoing necrosis and wherein the effective amount of alpha-1-antitrypsin inhibits the necrosis within the tissue.

2. The use of claim 1, wherein said effective amount is between 20 to 500 mg/kg/day of body weight.

3. The use of claim 1, wherein said medicament comprises a pharmaceutically acceptable carrier.

4. The use of claim 1, wherein said medicament further comprises at least a second therapeutic compound selected from the group comprising: an antibiotic, a beta blocker, an analgesic, or any combination thereof.

5. The use of claim 1, wherein the tissue undergoing necrosis is associated with a disease refractory to an anti-inflammatory agent.

6. The use of claim 1, wherein the tissue undergoing necrosis is associated with a disease selected from the group consisting of a cancer, a neurodegenerative disease, myocardial infarction, stroke, sepsis, ischemia, a liver disease, an open wound, or gangrene.

7. The use of claim 1, wherein the tissue undergoing necrosis is associated with AIDS.

8. The use of claim 1, wherein the tissue undergoing necrosis is associated with a disease of an immunocompromised subject.

9. The use of claim 1, wherein the tissue undergoing necrosis is associated with venom poisoning.

10. The use of claim 1, wherein said treatment is a prophylactic treatment.

11. A method for inhibiting necrosis in a tissue culture, said method comprising contacting a tissue culture **characterized by** undergoing necrosis with an amount of alpha-1-antitrypsin sufficient to inhibit necrosis in said tissue culture.

12. A method for preventing necrosis in a tissue culture, said method comprises contacting a tissue culture **characterized by** undergoing necrosis with an amount of alpha-1-antitrypsin sufficient to prevent necrosis in said tissue culture.

13. The use of claim 1, wherein the tissue undergoing necrosis is associated with pancreatitis.

14. The use of claim 13, said use results in reducing the symptoms associated with pancreatitis.

15. The use of claim 13, said use results in reducing the severity of pancreatitis.

16. The use of claim 13, said use results in ameliorating pancreatitis of said subject.

17. The use of claim 13, said use results in curing pancreatitis of said subject.

18. The use of claim 13, said use results in improving the wellbeing of said subject.

19. The use of claim 13, wherein said effective amount of said alpha-1-antitrypsin is between 20 to 100 mg/kg body weight/day.

## Patentansprüche

1. Verwendung einer therapeutisch wirksamen Menge von Alpha-1-Antitrypsin oder eines aktiven Homologs oder einer aktiven Variante davon zur Herstellung eines Medikaments für die Behandlung von Gewebenekrose, worin das Medikament an ein Gewebe verabreicht werden soll, das **dadurch gekennzeichnet ist, dass** es eine Nekrose erleidet, und worin die wirksame Menge von Alpha-1-Antitrypsin die Nekrose innerhalb des Gewebes hemmt.

2. Die Verwendung gemäß Anspruch 1, worin die wirksame Menge zwischen 20 bis 500 mg/kg/Tag des Körpergewichts beträgt.

3. Die Verwendung gemäß Anspruch 1, worin das Medikament einen pharmazeutisch verträglichen Träger umfasst.

4. Die Verwendung gemäß Anspruch 1, worin das Medikament weiter mindestens eine zweite therapeutische Verbindung umfasst, gewählt aus der Gruppe, die Folgendes umfasst: ein Antibiotikum, einen Betablocker, ein Analgetikum oder eine beliebige Kombination davon.

5. Die Verwendung gemäß Anspruch 1, worin das Gewebe, das eine Nekrose erleidet, mit einer Erkrankung verknüpft ist, die refraktär gegenüber einem Antiphlogistikum ist.

6. Die Verwendung gemäß Anspruch 1, worin das Gewebe, das eine Nekrose erleidet, mit einer Erkrankung verknüpft ist, die gewählt ist aus der Gruppe bestehend aus einer Krebserkrankung, einer neurodegenerativen Erkrankung, Myokardinfarkt, Schlaganfall, Sepsis, Ischämie, einer Lebererkrankung, einer offenen Wunde oder Gangrän.

7. Die Verwendung gemäß Anspruch 1, worin das Gewebe, das eine Nekrose erleidet, mit AIDS zusammenhängt.

8. Die Verwendung gemäß Anspruch 1, worin das Gewebe, das eine Nekrose erleidet, mit einer Erkrankung eines immunsupprimierten Individuums zusammenhängt.

9. Die Verwendung gemäß Anspruch 1, worin das Gewebe, das eine Nekrose erleidet, mit einer Vergiftung durch tierisches Gift zusammenhängt.

10. Die Verwendung gemäß Anspruch 1, worin die Behandlung eine prophylaktische Behandlung ist.

11. Ein Verfahren zur Hemmung von Nekrose in einer Gewebekultur, wobei das Verfahren Folgendes umfasst: das In-Kontakt-Bringen einer Gewebekultur, die **dadurch gekennzeichnet ist, dass** sie eine Nekrose erleidet, mit einer Menge an Alpha-1-Antitrypsin, die ausreicht, um die Nekrose in der Gewebekultur zu hemmen.

12. Ein Verfahren zur Prävention von Nekrose in einer Gewebekultur, wobei das Verfahren Folgendes umfasst: das In-Kontakt-Bringen einer Gewebekultur, die **dadurch gekennzeichnet ist, dass** sie eine Nekrose erleidet, mit einer Menge an Alpha-1-Antitrypsin, die ausreicht, um der Nekrose in der Gewebekultur vorzubeugen.

13. Die Verwendung gemäß Anspruch 1, worin das Gewebe, das eine Nekrose erleidet, mit Pankreatitis zusammenhängt.

14. Die Verwendung gemäß Anspruch 13; die Verwendung resultiert in der Minderung der mit Pankreatitis zusammenhängenden Symptome.

15. Die Verwendung gemäß Anspruch 13; die Verwendung resultiert in der Minderung der Schwere von Pankreatitis.

16. Die Verwendung gemäß Anspruch 13; die Verwendung resultiert in einer Besserung der Pankreatitis des Individuums.

17. Die Verwendung gemäß Anspruch 13; die Verwendung resultiert in der Heilung der Pankreatitis des Individuums.

18. Die Verwendung gemäß Anspruch 13; die Verwendung resultiert in der Besserung des Wohlbefindens des Individuums.

19. Die Verwendung gemäß Anspruch 13, wobei die wirksame Menge des Alpha-1-Antitrypsins zwischen 20 bis 100 mg/kg Körpergewicht/Tag liegt.

## Revendications

1. Utilisation d'une quantité thérapeutiquement efficace d'alpha-1-antitrypsine ou d'un homologue actif ou d'une variante active de celui-ci pour la préparation d'un médicament pour le traitement de la nécrose tissulaire, dans laquelle ledit médicament doit être administré à un tissu **caractérisé par** subir une nécrose et dans laquelle la quantité efficace de alpha-1-antitrypsine inhibe la nécrose dans le tissu.

2. Utilisation selon la revendication 1, dans laquelle ladite quantité efficace est comprise entre 20 et 500 mg / kg / jour de poids corporel.

3. Utilisation selon la revendication 1, dans laquelle ledit médicament comprend un support pharmaceutiquement acceptable.

4. Utilisation selon la revendication 1, dans laquelle ledit médicament comprend en outre au moins un second composé thérapeutique sélectionné parmi le groupe comprenant : un antibiotique, un bêta-bloquant, un analgésique, ou une combinaison quelconque de ceux-ci.

5. Utilisation selon la revendication 1, dans laquelle le tissu subissant une nécrose est associé à une maladie réfractaire à un agent anti-inflammatoire.

6. Utilisation selon la revendication 1, dans laquelle le tissu subissant une nécrose est associé à une maladie sélectionnée parmi le groupe consistant en un cancer, une maladie neurodégénérative, un infarctus du myocarde, un accident vasculaire cérébral, une septicémie, une ischémie, une maladie du foie, une plaie ouverte, ou une gangrène.

7. Utilisation selon la revendication 1, dans laquelle le tissu subissant une nécrose est associé au SIDA.

8. Utilisation selon la revendication 1, dans laquelle le tissu subissant une nécrose est associé à une maladie d'un sujet immunodéprimé.

9. Utilisation selon la revendication 1, dans laquelle le tissu subissant une nécrose est associé à un empoisonnement par venin.

10. Utilisation selon la revendication 1, dans laquelle ledit traitement est un traitement prophylactique.

11. Procédé pour inhiber la nécrose dans une culture tissulaire, ledit procédé comprenant la mise en contact d'une culture tissulaire **caractérisé par** une nécrose avec une quantité d'alpha-1-antitrypsine suffisante pour inhiber la nécrose dans ladite culture tissulaire.

12. Procédé pour empêcher la nécrose dans une culture tissulaire, ledit procédé comprenant la mise en contact d'une culture tissulaire **caractérisé par** subir une nécrose avec une quantité d'alpha-1-antitrypsine suffisante pour empêcher la nécrose dans ladite culture tissulaire.

13. Utilisation selon la revendication 1, dans laquelle le tissu subissant une nécrose est associé à une pancréatite.

14. Utilisation selon la revendication 13, ladite utilisation conduit à la réduction des symptômes associés à la pancréatite.

15. Utilisation selon la revendication 13, ladite utilisation conduit à la réduction de la sévérité de la pancréatite.

16. Utilisation selon la revendication 13, ladite utilisation conduit à l'amélioration de la pancréatite dudit sujet.

17. Utilisation selon la revendication 13, ladite utilisation conduit à la guérison de la pancréatite dudit sujet.

18. Utilisation selon la revendication 13, ladite utilisation conduit à l'amélioration du bien-être dudit sujet.

19. Utilisation selon la revendication 13, dans laquelle ladite quantité efficace de ladite alpha-1-antitrypsine est comprise entre 20 et 100 mg / kg de poids corporel / jour.
